# EUROPÄISCHE PATENTSCHRIFT

(11) **EP 0 371 361 B1**
(45) Veröffentlichungstag und Bekanntmachung des Hinweises auf die Patenterteilung: **02.03.1994**
(21) Anmeldenummer: 89121380.3
(22) Anmeldetag: 18.11.1989
(51) Int. Cl.: C07D 231/00, A61K 31/00

(54) **Verfahren zur Herstellung von 4-Chlorpyrazolen**
Process for the preparation of 4-chlorpyrazoles
Procédé de préparation de 4-chlorpyrazoles

(30) Priorität: 30.11.1988 DE 3840342
(43) Veröffentlichungstag der Anmeldung: 06.06.1990
(73) Patentinhaber: BASF Aktiengesellschaft, 67063 Ludwigshafen (DE)
(72) Erfinder: Baus, Ulf, Dr., D-6915 Dossenheim (DE); Reuther, Wolfgang, Dr., D-6900 Heidelberg (DE)

(56) Entgegenhaltungen:
- ALAN R. KATRITZKY, CHARLES W. REES: "Comprehensive heterocyclic chemistry", Band 5, Teil 4A, p. 240, 1984, Pergamon Press, Oxford, GB
- JOURNAL OF ORGANIC CHEMISTRY, Band 42, Nr. 23, 11. November 1977, Seiten 3721-3724, Easton, US; JEREMIAH P. FREEMAN et al.: "Synthesis and Some Reactions of 4H-Pyrazole Derivatives"
- Liebigs Annalen der Chemie, Band 598, Heft 3, Seiten 186-197, 1956; Hüttel et al "Die Chlorierung der Pyrazole"

## Beschreibung

Die Erfindung betrifft ein verbessertes Verfahren zur Herstellung von 4-Chlorpyrazolen aus Pyrazolen.

Aus J. Org. Chem. 42, 3721 bis 3724 (1977) ist ein Verfahren zur Herstellung von 4-Chlor-4H-pyrazol-1-monoxiden oder - 1,2-dioxiden bekannt.

Aus Comprehensive Heterocyclic Chemistry Band 5, Teil 4A, Seite 240 (1984) ist ein Verfahren zur Herstellung von 4-Chlor-3-methyl-1-arylpyrazol durch Chlorierung mit Thionylchlorid in Chloroform bekannt.

Aus Liebigs Ann. Chem., Bd. 598 (1956), 186-197 ist die Umsetzung von Pyrazol unter Zusatz von Essigsäure mit einer 9 %igen Lösung von NaOCl zu 4-Chlorpyrazol mit 70 %iger Ausbeute bekannt. Der Nachteil dieses Verfahrens liegt in der Verwendung von Essigsäure und der nicht befriedigenden Ausbeute.

Der Erfindung lag daher die Aufgabe zugrunde, ein verbessertes Verfahren zur Herstellung von 4-Chlorpyrazolen zu finden und den Nachteilen abzuhelfen.

Demgemäß wurde ein verbessertes Verfahren zur Herstellung von 4-Chlorpyrazolen der allgemeinen Formel I 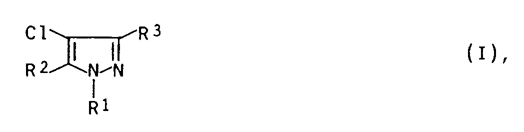
in der R¹ bis R³ unabhängig voneinander Wasserstoff oder einen unter den Reaktionsbedingungen inerten Rest bedeuten, gefunden, welches dadurch gekennzeichnet ist, daß man Pyrazole der allgemeinen Formel II 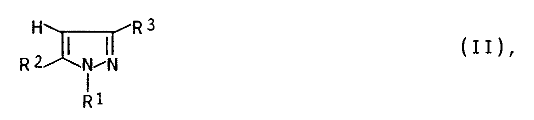
in der R¹ bis R³ die obengenannten Bedeutungen haben, mit 0,95 bis 10 Equivalenten Hypochlorsäure oder deren Salzen in Abwesenheit einer Carbonsäure umsetzt.

Die 4-Chlorpyrazole I sind nach folgender Methode erhältlich:
Die Umsetzung erfolgt zwischen einem in 4-Stellung Wasserstoff tragenden Pyrazol II und Hypochlorsäure oder deren Salzen bei Temperaturen von (-20) bis +70°C in Abwesenheit einer Carbonsäure in einem geeigneten Lösungsmittel nach folgender Reaktionsgleichung: 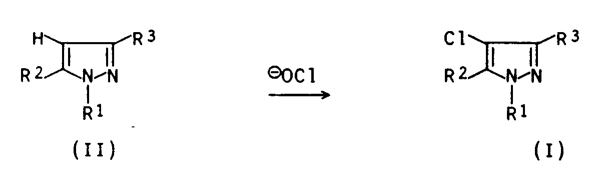
Die Umsetzung wird vorzugsweise bei 0 bis 40°C, besonders bevorzugt bei 5 bis 30°C durchgeführt.

Die Verbindungen der Formel II sind z.T. aus Liebigs Ann. Chem., Bd. 598 (1956), 186-197 bekannt oder können nach den dort oder in der DE-A-16 70 060 beschriebenen verfahren hergestellt werden.

Die Hypochlorsäure oder deren Salze werden in 0,95 bis 10 Equivalenten bezogen auf die Verbindungen II eingesetzt, bevorzugt 0,99 bis 2, besonders bevorzugt 1 bis 1,5, d.h. das Molverhältnis zwischen Hypochlorsäure oder deren Salzen und den Verbindungen II beträgt 0,95:1 bis 10:1, vorzugsweise 0,99:1 bis 2:1, besonders bevorzugt 1:1 bis 1,5:1.

Geeignete Lösungsmittel für die Reaktion sind Ketone wie Acetone, acyclische und cyclische Ether wie Tetrahydrofuran, Glykole wie Ethylenglykol und Propylenglykol oder Glykolether wie Diglym auch in Mischungen mit Wasser, bevorzugt ist Wasser.

Bei der Aufarbeitung wird die Reaktionsmischung auf einen pH-Wert von 7 bis 14, vorzugsweise von 8 bis 12, besonders bevorzugt von 9 bis 11 gebracht.

Zur Extraktion eignen sich Ether wie Diethylether und Methyl-tert.-butylether, Ester wie Essigsäuremethylester und Essigsäureethylester, aromatische Kohlenwasserstoffe wie Benzol, Toluol und die Xylole, chlorierte Kohlenwasserstoffe wie Methylenchlorid und Chloroform oder deren Mischungen, bevorzugt sind Ester und Ether wie oben genannt.

Die Substituenten R¹ bis R³ in den Formeln I und II bedeuten unabhängig voneinander Wasserstoff oder einen unter den Reaktionsbedingungen inerten Rest. Solche Reste sind C₁- bis C₈-Alkyl wie Methyl, Ethyl, n-Propyl, iso-Propyl, n-Butyl, iso-Butyl, sec.-Butyl, tert.-Butyl, Pentyl, Hexyl, Heptyl und Octyl, C₁- bis C₄-Halogenalkyl wie C₁- bis C₄-Chlor- und/oder Fluoralkyl, z.B. Fluormethyl, Difluormethyl, Trifluormethyl, Chlormethyl, Dichlormethyl, Trichlormethyl, Chlor-fluormethyl, 2,2,2-Trifluorethyl und 2,2,2-Trichlorethyl, bevorzugt sind Methyl und Ethyl.

Unter den Verbindungen I und II sind folgende bevorzugt:
4-Chlorpyrazol,
4-Chlor-N-methylpyrazol,
4-Chlor-3,5-dimethylpyrazol,
4-Chlor-3-methylpyrazol,
3-Methylpyrazol,
Pyrazol,
N-Methylpyrazol,
3,5-Dimethylpyrazol.

4-Chlorpyrazole sind Zwischenprodukte für die Herstellung von Wirkstoffen, z.B.von Biociden (DE-A-34 12 080). 4-Chlorpyrazol kann z.B. als pharmazeutischer Wirkstoff bei der Kontrolle epileptischer Erkrankungen eingesetzt werden.

### Beispiele

### Beispiel 1

34 g (0,5 mol) Pyrazol wurden in 100 ml Wasser suspendiert. Unter ständigem Rühren wurden 425 g (0,5 mol) einer wäßrigen 8,7 gew.%igen NaOCl-Lösung so zugetropft, daß die Temperatur der Reaktionsmischung nicht über 30°C stieg. Die Reaktion wurde mit HPLC-Analyse verfolgt. Nach beendeter Reaktion wurde 35 %ige Schwefelsäure zugegeben und die Mischung bei pH 11 mit 300 ml Essigester extrahiert. Nach Trocknen der vereinigten org. Phasen und Entfernen des Lösungsmittels i.Vak. kristallisierte 4-Chlorpyrazol als schwach gelber Feststoff. Ausbeute: 51 g (0,5 mol, 99 %).

| Elementaranalyse des Rohproduktes: | | | | |
|---|---|---|---|---|
| ber.: | C 35,1 | H 2,9 | N 27,3 | Cl 34,5 |
| gef.: | C 35,2 | H 3,4 | N 26,9 | Cl 33,6 |

## Patentansprüche

1. Verfahren zur Herstellung von 4-Chlorpyrazolen der allgemeinen Formel I 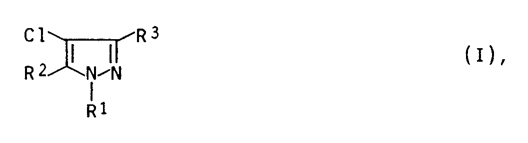 in der R¹ bis R³ unabhängig voneinander Wasserstoff oder einen unter den verfahrensgemäßen Reaktionsbedingungen inerten Rest bedeuten, durch Umsetzung von Pyrazolen der allgemeinen Formel II 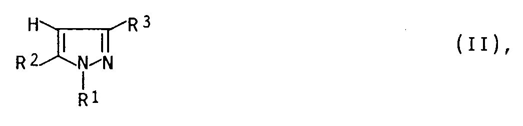 in der R¹ bis R³ die obengenannten Bedeutungen haben, mit 0,95 bis 10 Equivalenten Hypochlorsäure oder deren Salzen in wäßrigem Medium, dadurch gekennzeichnet, daß man in Abwesenheit einer Carbonsäure bei Temperaturen von (-20) bis +70°C arbeitet.

2. Verfahren nach Anspruch 1, dadurch gekennzeichnet, daß man die Umsetzung in wäßrigem Medium vornimmt und die Isolierung bei pH-Werten von 7 bis 14 durchführt.

3. Verfahren nach Anspruch 1, dadurch gekennzeichnet, daß man die Umsetzung in wäßrigem Medium vornimmt und die Isolierung bei pH-Werten von 8 bis 12 durchführt.

## Claims

1. A process for preparing a 4-chloropyrazole of the general formula I 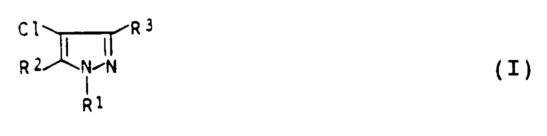 where R¹, R² and R³ are each independently of the others hydrogen or a radical which is inert under the reaction conditions of the process, by reacting a pyrazole of the general formula II 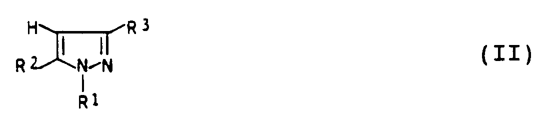 where R¹, R² and R³ are each as defined above, with 0.95-10 equivalents of hypochloric acid, or a salt thereof, in an aqueous medium, which comprises working at from (-20) to + 70°C in the absence of any carboxylic acid.

2. A process as claimed in claim 1, wherein the reaction in an aqueous medium is followed by isolation at pH 7-14.

3. A process as claimed in claim 1, wherein the reaction in an aqueous medium is followed by isolation at pH 8-12.

## Revendications

1. Procédé de préparation de 4-chloropyrazoles de formule générale I 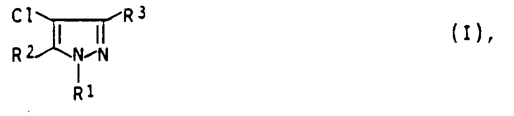 dans laquelle R¹ à R³ représentent chacun, indépendamment les uns des autres, un atome d'hydrogène ou un reste inerte dans les conditions de la réaction selon le procédé, par réaction, en milieu aqueux, de pyrazoles de formule générale II 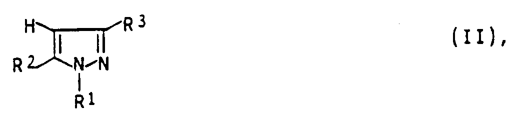 dans laquelle R¹ à R³ ont les significations susmentionnées, avec 0,95 à 10 équivalents d'acide hypochloreux ou de ses sels, caractérisé en ce qu'on procède en l'absence d'un acide carboxylique à des températures de (-20) à +70°C.

2. Procédé selon la revendication 1, caractérisé en ce qu'on conduit la réaction en milieu aqueux et on effectue l'isolement à un pH de 7 à 14.

3. Procédé selon la revendication 1, caractérisé en ce qu'on conduit la réaction en milieu aqueux et on effectue l'isolement à un pH de 8 à 12.
